# EUROPEAN PATENT SPECIFICATION

(11) **EP 1 327 148 B1**
(45) Date of publication and mention of the grant of the patent: **15.04.2009**
(21) Application number: 01977793.7
(22) Date of filing: 19.09.2001
(51) Int. Cl.: G01N 33/564

(54) **PATTERN RECOGNITION METHOD FOR DIAGNOSIS OF SYSTEMIC AUTOIMMUNE DISEASES**
MUSTERERKENNUNGSVERFAHREN ZUR DIAGNOSE VON SYSTEMISCHEN AUTOIMMUNERKRANKUNGEN
PROCEDE DE RECONNAISSANCE DE FORMES PERMETTANT LE DIAGNOSTIC DE MALADIES AUTO-IMMUNES SYSTEMIQUES

(30) Priority: 17.10.2000 US 691405
(43) Date of publication of application: 16.07.2003
(73) Proprietor: BIO-RAD LABORATORIES, INC., Hercules California 94547 (US)
(72) Inventor: BINDER, Steven, R., Berkeley, CA 94708 (US); GLOSSENGER, John, Benicia, CA 94510 (US)
(74) Representative: Arends, William Gerrit
(86) International application number: PCT/US2001/042213
(87) International publication number: WO 2002/033415

(56) References cited:
- WO-A-96/12187
- US-A- 5 674 688
- THOMPSON D ET AL: "The clinical significance of autoantibody profiles in patients with systemic lupus erythematosus." LUPUS. ENGLAND FEB 1993, vol. 2, no. 1, February 1993 (1993-02), pages 15-19, XP002218843 ISSN: 0961-2033
- GRUS FRANZ-HERMANN ET AL: "Identification and classification of autoantibody repertoires (Western blots) with a pattern recognition algorithm by an artificial neural network." ELECTROPHORESIS, vol. 18, no. 7, 1997, pages 1120-1125, XP002218844 ISSN: 0173-0835
- PETROVECKI M ET AL: "An algorithm for leukaemia immunophenotype pattern recognition" MEDICAL INFORMATICS, JAN.-MARCH 1993, UK, vol. 18, no. 1, pages 11-21, XP002218845 ISSN: 0307-7640

## Description

### BACKGROUND OF THE INVENTION

### 1. Field of the Invention

This invention arises in the field of autoimmune diseases and diagnostic methods for these diseases. The invention also relates to statistical methods of data analysis and their application to immunodiagnostics.

### 2. Description of the Prior Art

Autoimmune diseases are conditions in which the immune system attacks cells, tissues, and organs of one's own body rather than bacteria, viruses, and other microbes that invade the body from the outside. There are many different autoimmune diseases, attacking different parts of the body ranging from the gut to the brain, for example, or from the vascular system to the skin, and the attacks occur in different ways. Some autoimmune diseases are tissue- or organ-specific, while others affect several tissues. Diseases of this latter category are termed "systemic autoimmune diseases," and the symptoms may vary from one patient to the next, with tissue injury and inflammation occurring in multiple sites in organs without relation to their antigenic makeup. Examples of systemic autoimmune diseases are rheumatoid arthritis, systemic lupus erythmatosus, scleroderma, polymyositis, dermatomyositis, Sjögren's syndrome, and spondyloarthropathies such as ankylosing spondylitis. Autoimmune diseases are generally multifactorial in origin, with some of the contributing factors being genetic disposition, host factors (such as T cell defects and polyclonal stimulation of B cells that are resistant to controls), environmental factors (such as certain microbial infections), and antigen-driven mechanisms (such as sequestered antigens or cross-reacting exogenous antigens).

Due to their overlapping symptoms and complex etiologies, autoimmune diseases are difficult to diagnose. Attempts at diagnosis are generally based on symptoms, together with the findings from a physical examination and results obtained from laboratory tests. Symptoms of many autoimmune diseases are nonspecific, and while laboratory test results may help they are often inadequate to confirm a diagnosis, particularly in the early phases of the disease. The problem is aggravated when the symptoms are transient and the laboratory results are inconclusive, as they are in many cases. For some autoimmune diseases, the patient will respond completely to treatment if the disease is identified at an early stage. Often, however, a specific diagnosis cannot be made until the disease is in an advanced state. The problem is particularly acute with systemic autoimmune diseases.

It is commonly believed that the presence of autoimmune antibody is indicative of an autoimmune disease. This belief is disproved however by the fact that almost all antibodies are present in measurable amounts in any individual, and comparisons with reference levels from healthy individuals are frustrated by the wide variations in normal antibody levels due to demographics such as gender, age, geographical region of domicile, nationality and race.. In addition, the number of differentiable antibodies that must be investigated in the diagnosis is large, which presents an often unmanageable burden on the clinical laboratory in its attempts to obtain a specific and reasonably accurate diagnosis.

The most widely used method of identifying a systemic autoimmune disease is indirect immunofluorescence, a manual method that requires a well trained technician. The result appears as a pattern of distinctive characteristics that are seen on cells (typically HeLa cells) that have been fixed on a slide, treated with serum, washed and then labeled. The distinctive characteristics of the pattern are a relatively speckled, relatively homogeneous appearance, or the like, and diagnosis is achieved by comparing the pattern with those of individuals with known diseases. The most common patterns are associated with several diseases, however, which obscures the diagnosis. As a result, additional testing for specific antibodies is needed. Even then, an experienced immunologist or rheumatologist must be called upon to interpret the results and make the final diagnosis.

Recently, enzyme-linked immunosorbent assays (ELISAs) have become available for preliminary screening of patients with symptoms suggesting an autoimmune disease. Specimens reading positive in the screening assay are then submitted for a specific diagnostic assay to determine which particular autoantibody is present and therefore which specific disease is suggested. Many autoimmune disease patients, however, suffer from two or more different autoimmune diseases, and this confounds efforts at achieving an accurate diagnosis when using this diagnostic method.

### SUMMARY OF THE INVENTION

The difficulties enumerated above and others are addressed by the present invention, which resides in a computer inplemented method as defined in claim 1. Thus a particular systemic autoimmune disease (or diseases) is identifed by obtaining multianalyte test data from a patient suspected of suffering from a systemic autoimmune disease, applying a statistical pattern recognition method to this data to compare it with a multitude of reference data sets, and using the comparison to identify the specific disease(s) that the patient is suffering from. With the aid of computer software, the pattern recognition method processes the entire pattern of results in a medical decision support system. Applying pattern recognition methods in this manner permits the clinician to use test results from a single biological sample obtained from the patient, and to obtain both a diagnosis of the disease(s) and an assessment of the confidence level of the diagnosis. The autoantibodies selected can be those that are known to be frequently elevated in various systemic autoimmune diseases, and particular antibodies can be included or omitted from the set in accordance with their perceived relevance to the particular disease or group of possible diseases. Two or more diseases can be diagnosed simultaneously, and confidence levels assigned to each, without the need for separate samples or analyses.

The method of this invention offers a number of advantages over diagnostic methods currently known. One advantage is the elimination of the need for a manual assay, since the multianalyte assay itself can be performed by automated instrumentation. Another is the ability to obtain a diagnosis without the need for screening followed by confirmatory tests. In addition, the invention provides a diagnosis among a large number of possible diseases with a single-step sample analysis. Further, the invention eliminates the need for the intervention of a skilled professional to obtain an interpretation of the result. The result can therefore be transmitted directly to the ordering physician, even if that physician is a generalist and unfamiliar with the autoantibodies being measured.

These and other objects, advantages, features and embodiments of the invention will be apparent from the description that follows.

### DETAILED DESCRIPTION OF THE INVENTION

### AND SPECIFIC EMBODIMENTS

Pattern recognition systems for use in the practice of this invention typically begin with the development of a "training set," a term understood in the art of statistical data analysis to mean data from a set of samples from reliable ("pedigreed") sources. The set will include samples having disease conditions that are known from a previous and independent diagnosis as well as samples that are disease-free. The method in accordance with this invention thus begins with reference samples from a series of subjects each of whom is known to have, or to have had, a particular systemic autoimmune disease, as well as a series of subjects each of whom is known to be disease-free. This training set includes the full scope of the systemic autoimmune diseases sought to be investigated for a particular patient or for patients in general. Multianalyte analyses are performed on each sample in the training set, and the results are entered into a database in a manner resembling a multi-dimensional array in which each sample can be thought of as a point in a multi-dimensional space. The coordinates of the point that define its location in the space represent the value of the test results, one coordinate for each test. As a simple case, if the number of tests performed per sample in the training set is only two, the training set points will be arranged in a two-dimensional (*x*, *y*) plot, where the horizontal (*x*) axis corresponds to one of the tests and the vertical (*y*) axis corresponds to the other. In addition to its location in the two-dimensional plot (i.e., the *x* and *y* values) which is determined by the test results, each point is labeled as to the particular disease that is associated with its source. The corresponding array for a three-test system is a three-dimensional plot with orthogonal *x*, *y*, and *z* axes. Corresponding arrays for systems involving four or more tests are not capable of visualization, but are established in an analogous manner.

Once the training set is established and its test data entered into the database, the system is ready to receive data from a single patient sample or from a succession of patient samples with no need to recreate the database, or to re-analyze the training set, or to select and analyze a different training set. The patient sample is subjected to the same tests as the samples in the training set, and these test results are inserted into the database. Using the illustrative representational description of the preceding paragraph, the patient sample test results are added to the space as a point at a location defined by coordinates equal to the test values.

The determination of particular diseases is then achieved by a statistical comparison between the values of the patient sample test results and those of the training set. The statistical analysis used for the comparison is k-nearest neighbor analysis. While this method is known in the art, the following explanation will assist the reader in understanding how the algorithm is applied in the practice of the present invention.

The k-nearest neighbor algorithm reads the data from the patient sample into memory which also contains the database of the training set. The algorithm then calculates the numeric distances from the patient's test point to the data points in the memory from the training set that are in the vicinity of the patient's test point, and from those points selects the *k* whose distances are the shortest. Thus, if *k* is 15, the algorithm selects the 15 that are the shortest distances from the patient's test point. The disease associated with the *k* nearest data points is then identified as that which is present in the patient sample, and if the *k* points are associated with more than one disease, the diagnosis is for each of the diseases indicated. A refinement for the algorithm is one in which the distance values of the various points are compared, and when the values for points representing two different diseases differ by less than a minimum difference, both diseases are considered to be equally likely. In a further refinement, the algorithm processes the numerical values of the distances to determine a confidence level. This can be done by using "similarity" values between the patient test results and each of the *k* data points and assigning a relative value to each disease by dividing the similarity for that disease by the sum of similarities. The final diagnosis is generally selected as that disease (or those diseases) with the highest relative value. In general, the k-nearest neighbor algorithm is a "case-oriented" system, or one that compares the patient's test data to the data from other individuals for whom demographic and other personal information is available in addition to the disease, such as age, sex, the length of time that the individual has had the disease, and similar factors. This information permits a diagnosing physician to exercise individual judgment as to whether or not to use the patients proposed by the algorithm as reference points for the diagnosis.

The technique and its application in this context are computer implemented. Software specifically designed for particular analyses is readily developed and a routine matter to the skilled software engineer.

The test data used in the present invention are values that are proportional to, or otherwise representative of, the levels of various antibodies that are associated to various degrees with systemic autoimmune diseases. Currently, over 100 antibodies are known to be expressed in autoimmune diseases. Examples are listed in Peter, J.B., et al., Autoantibodies, Elsevier Science B.V., Amsterdam (1996).

The antigens to many of these antibodies are commercially available, while the antigens to others are readily synthesized based on descriptions of them that are available in the literature. Some of the sources of these antigens are BiosPacific, of Emeryville, California, USA; Immunovision, of Springdale, Arkansas, USA; and KMI Diagnostics, Inc., of Minneapolis, Minnesota, USA. Examples of the antibodies that are expressed in autoimmune diseases, identified by the antigens to which they bind in an immunoassay, are listed below:
SSA 60
SSA 60
SSA 52
SSB 48
Sm BB'
Sm D1
RNP 68
RNP A
RNP C
Fibrillarin
Riboproteins P0, P1, and P2
dsDNA
Nucleosome
Ku
Centromere A
Centromere B
Scl-70
Pm-Scl
RNA-Polymerases 1,2, and 3
Th
Jo-1
Mi-2
PL7
PL12
SRP

The present invention is not intended to be limited to antibodies identified by the antigens in the above list. Instead, the number of antibodies that are detected and quantitated from the training set, and the number of antibodies detected and quantitated in the patient sample as well, may vary and are not critical to this invention. The particular antibodies selected and the number of such antibodies in the selected group will however affect the accuracy of the assay and its ability to identify the systemic autoimmune disease(s) present. In most cases, it is contemplated that from 10 to 100 antibodies will be used, preferably from 15 to 25. In preferred embodiments of the invention, at least 15 of the antibodies identified by the antigens in the above list will be used, and in further preferred embodiments, all of the antibodies in the above list will be used.

Similarly, the number of reference samples of different sources used to develop the training set may vary as well and is not critical to this invention, although the number selected may affect the range of autoimmune diseases that can be detected. In most cases, it is contemplated that 100 to 10,000 reference samples will be used, preferably from 200 to 2000.

The quantitative levels of each antibody are determinable by conventional antibody assays. Immunoassays are generally preferred. Both antigen capture (indirect immunoassays) and class capture assays can be used, antigen capture being preferred, and detection can be achieved by enzyme labels, radioactive labels, or fluorescent labels. The assays may be colorimetric, luminometric, fluorometric, enzymetric, radiometric, or other methods such as nephelometry, turbidimetry, particle counting, or visual assessment. Examples of enzyme labels are alkaline phosphatase, β-D-galactosidase, glucose-6-phosphate dehydrogenase, horseradish peroxidase, β-lactamase, melittin, and urease. Examples of radioisotopic labels are cobalt-57 and iodine-125. Examples of fluorescent labels are succinimidyl esters and vinyl sulfones of xanthenes, cyanines, coumarins, benzimides, phenanthridines, ethidium dyes, acridine dyes, carbazole dyes, phenoxazine dyes; porphyrin dyes, quinoline dyes, and naturally occurring protein dyes. Fluoresceins and rhodamines are particular types of xanthene dyes. Specific examples are 6-carboxyfluorescein, 6-carboxy-4',5'-dichloro-2',7'-dimethoxyfluorescein, N,N,N',N'-tetramethyl-6-carboxyrhodamine, 6-carboxy-X-rhodamine, 5-carboxyrhodamine-6G, 5-carboxyrhodamine-6G, tetramethylrhodamine, Rhodamine Green, and Rhodamine Red. Umbelliferone is an example of a coumarin. Hoechst 33258 is an example of a benzimide dye. Texas Red is an example of a phenanthridine dye. Examples of cyanine succinimidyl ester dyes are sulfoindocyanine succinimidyl esters, (carboxyalkyl)cyanines succinimidyl esters, and BODIPY succinimidyl esters (Molecular Probes, Inc., Eugene, Oregon, USA). Examples of naturally occurring protein dyes are phycoerythrins. Many other examples will be readily apparent to those skilled in the art.

The sequence and manner of performing quantitative assays of multiple antibodies in the practice of this invention may vary widely. In preferred embodiments, the assays are performed simultaneously in a single sample by a multiplexed system that differentiates the assays from each other and thus provides individual values for each of the antibodies. This may be achieved in a variety of ways. Heterogeneous binding assays, in which one of the binding members is immobilized on a solid phase, are the most efficient, since they provide a ready means for separating the bound pairs from unbound species. Multiplexed heterogeneous binding assays in which solid particles, preferably magnetic microbeads, are used as the solid phase, are illustrative examples. In these assays, the beads are sorted into groups, each group containing the reagents for a single assay covalently bonded to the bead surface, the various groups differentiable from one another by virtue of distinguishing characteristics that permit separate detection of the assay result in one group from those in the other groups. The differentiating parameter may be particle size, fluorescence (independent of the fluorescent label used in the assay, when fluorescent assay labels are in fact used), light scatter, light emission, or absorbance. The binding member that is covalently bonded to the bead surface will vary depending on the type of immunoassay that is being used. Since the analytes in accordance with this invention are all antibodies, the antigens by which the antibodies are defined serve as particularly convenient binding members bonded to the bead surface. Actual differentiation of the bead groups is readily achieved by flow cytometry.

When magnetic beads are used, the magnetic character permits quick separation of the solid and liquid phases and convenient washing of the solid phase. A magnetic character can be imparted to the beads by using beads made of paramagnetic materials, ferromagnetic materials, ferrimagnetic materials, or metamagnetic materials. The magnetically responsive material is preferably only one component of the bead, the remainder consisting of a polymeric material to which the magnetically responsive material is affixed or otherwise combined. The polymeric material can be chemically derivatized to permit attachment of the antigen or other assay reagent that enters into the binding reaction.

Multiplex systems of this description as well as others useful in the practice of this invention are disclosed in pending United States patent application no. 09/302,920, filed April 30, 1999, entitled "Multiplex Flow Assays Preferably With Magnetic Particles as Solid Phase," Michael 1. Watkins et al., inventors.

The biological samples on which the tests are performed can be any biological fluid extracted from the patient that is likely to have a detectable antibody population that is characteristic of the disease(s) sought to be investigated. Serum, plasma, urine, or cerebrospinal fluid may be used. Serum samples are preferred.

This invention is used in the detection and diagnosis of the following systemic autoimmune diseases: systemic lupus erythmatosus (SLE), Sjögren's syndrome, scleroderma, polymyositis and dermatomyositis, CREST (*c*alcinosis, *R*aynaud's phenomenon, *e*sophageal involvement, *s*clerodactyly, and *t*elangiectasis), and mixed connective tissue disease.

The foregoing is offered primarily for purposes of illustration. Further modifications and variations of the various parameters of the composition and method of this invention will be readily apparent to those skilled in the art. For example, the pattern recognition algorithm can be adjusted to control the rate of false negative results, by setting a boundary that differentiates "healthy" from "unhealthy" for a particular disease at a higher level than the corresponding boundary for other diseases, thereby lowering the number of samples that will indicate the presence of the disease. These and other variations are included within the scope of the invention.

## Claims

1. A computer-implemented method for the identification of a systemic autoimmune disease in a test subject suspected of suffering from one or more otherwise unidentified systemic autoimmune diseases selected from the group consisting of systemic lupus erythematosus, scleroderma, Sjögren's syndrome, polymyositis, dermatomyositis, CREST, and mixed connective tissue disease, said method comprising:
(a) receiving a test data set for the test subject, wherein the test data set is obtained from a biological sample of the test subject and wherein the test data set includes values representing levels of a plurality of autoantibodies;
(b) comparing the test data set and a library of reference data sets by applying k-nearest neighbor analysis to produce a statistically derived decision indicating whether, out of the range of none, one and more than one of said systemic autoimmune disease, the test subject is suffering from none, one or more than one of said systemic autoimmune diseases, wherein the reference data sets have been obtained by storing a reference data sets including i) reference data sets obtained by subjecting biological samples of reference subjects, each known to have one of said one or more systemic autoimmune diseases, to a set of one or more tests, and ii) reference data sets obtained by subjecting biological samples of reference subjects known to not have one of said one or more systemic autoimmune diseases to said set of one or more tests, such that each stored reference data set includes values representing levels of the plurality of autoantibodies and each stored reference data set is associated with none, one, or more of said systemic autoimmune diseases; and
(c) identifying which of said systemic autoimmune diseases the test subject is suffering from if the statistically derived decision indicates that the test subject is suffering from one or more of said systemic autoimmune diseases.

2. A method in accordance with claim 1 in which said test subject is suffering from two systemic autoimmune diseases, and step (b) comprises applying k-nearest neighbor analysis to produce a statistically derived decision indicating which two systemic autoimmune diseases said test subject is suffering from.

3. A method in accordance with claim 1 in which said plurality of autoantibodies numbers from 10 to 100 autoantibodies.

4. A method in accordance with claim 1 in which said plurality of autoantibodies numbers from 15 to 25 autoantibodies.

5. A method in accordance with claim 1 in which said plurality of autoantibodies comprises antibodies to at least fifteen of the following antigens:
SSA 60
SSA 60
SSA 52
SSB 48
Sm BB¹
Sm D1
RNP 68
RNP A
RNP C
Fibrillarin
Riboproteins P0, P1, and P2
dsDNA
Nucleosome
Ku
Centromere A
Centromere B
Scl-70
Pm-Scl
RNA-Polymerases 1, 2, and 3
Th
Jo-1
Mi-2
PL7
PL12
SRP

6. A method in accordance with claim 1 in which said plurality of antoantibodies comprises antibodies to each of the following antigens:
SSA 60
SSA 60
SSA 52
SSB 48
Sm BB¹
Sm D1
RNP 68
RNP A
RNP C
Fibrillarin
Riboproteins P0, P1, and P2
dsDNA
Nucleosome
Ku
Centromere A
Centromere B
Scl-70
Pm-Scl
RNA-Polymerases 1, 2, and 3
Th
Jo-1
Mi-2
PL7
PL12
SRP

7. A method in accordance with claim 1 in which said library of reference data sets represents from 100 to 10,000 biological samples from reference subjects known to have systemic autoimmune diseases of known identity.

8. A method in accordance with claim 1 in which said library of reference data sets represents from 200 to 2000 biological samples from reference subjects known to have systemic autoimmune diseases of known identity.

9. A method in accordance with claim 1 in which step (b) further comprises assigning a confidence level to said determination.

10. A method in accordance with claim 1 in which said biological sample from said test subject is a member selected from the group consisting of serum, plasma, urine, and cerebrospinal fluid.

11. A method in accordance with claim 1 in which said biological sample from said test subject is serum.

12. A method in accordance with claim 1 in which step (a) is performed by immunoassay.

13. A method in accordance with claim 1 in which step (a) is performed by immunoassay with fluorescence detection.

14. A method in accordance with claim 1 in which one of said systemic autoimmune disease is systemic lupus erythematosus.

## Patentansprüche

1. Computerimplementiertes Verfahren zur Identifizierung einer systemischen Autoimmunerkrankung bei einer Testperson, von der vermutet wird, dass sie an einer oder mehreren sonst nicht identifizierten systemischen Autoimmunerkrankungen leidet, ausgewählt aus der Gruppe bestehend aus systemischer Lupus Erythematodes, Sclerodermie, Sjögren-Syndrom, Polymyositis, Dermatomyositis; CREST und mixed connective tissue desease (Sharp-Syndrom), wobei das Verfahren umfasst:
(a) Empfangen eines Testdatensatzes für die Testperson, wobei der Testdatensatz von einer biologischen Probe der Testperson erhalten ist und wobei der Testdatensatz Werte, die Niveaus von einer Mehrzahl von Autoantikörpern darstellen, einschließen;
(b) Vergleichen des Testdatensatzes und einer Bibliothek von Bezugsdatensätzen durch das Anwenden einer k-Nächster-Nachbar-Analyse, um eine statistisch abgeleitete Entscheidung zu erzeugen, die angibt, ob, aus dem Bereich von keiner, einer oder mehr als einer der systemischen Autoimmunerkrankungen, die Testpersonen an keiner, einer oder mehr als eine der systemischen Autoimmunerkrankungen leidet, und wobei die Bezugsdatensätze erhalten wurden durch speichern einer Mehrzahl von Bezugsdatensätzen einschließend i) Bezugsdatensätze erhalten durch das Unterwerfen von biologischen Proben der Bezugspersonen, wobei jedem bekannt ist, dass sie eine oder mehrere sytemische Autoimmunerkrankungen haben, an einen Satz von einem oder mehreren Tests, und ii) Bezugsdatensätze erhalten durch das Unterwerfen von biologischen Proben der Bezugspersonen, vom denen bekannt ist, dass sie keine der einen oder mehreren systemischen Autoimmunerkrankungen haben, an den Satz von einem oder mehreren Tests, so dass jeder gespeicherte Datensatz Werte enthält, die Niveaus von der Mehrzahl von Autoantikörper darstellen und jeder gespeicherte Bezugsdatensatz mit keiner, einer oder mehreren der systemischen Autoimmunerkrankungen assoziiert ist; und
(c) Identifizieren an welcher der systemischen Autoimmunerkrankungen die Testperson leidet, wenn die statistisch abgeleitete Entscheidung angibt, dass die Testperson an einer oder mehreren der sytemischen Autoimmunerkrankungen leidet.

2. Verfahren gemäß Anspruch 1, wobei die Testperson an zwei systemischen Autoimmunerkrankungen leidet, und Schritt (b) umfasst das Anwenden einer k-Nächster-Nachbar-Analyse, um eine statistisch abgeleitete Entscheidung zu erzeugen, die angibt, an welchen beiden systemischen Autoimmunerkrankungen die Testperson leidet.

3. Verfahren gemäß Anspruch 1, wobei die Mehrzahl von Antikörpern 10 bis 100 Antikörper zählt.

4. Verfahren gemäß Anspruch 1, wobei die Mehrzahl von Antikörpern 15 bis 25 Antikörper zählt.

5. Verfahren gemäß Anspruch 1, wobei die Mehrzahl von Antikörpern Antikörper gegen mindestens fünfzehn der folgenden Antigene umfasst:
SSA 60
SSA 60
SSA 52
SSB 48
Sm BB¹
Sm D1
RNP 68
RNP A
RNP C
Fibrillarin
Riboproteine P0, P 1 und P2
dsDNA
Nukleosom
Ku
Zentromer A
Zentromer B
Scl-70
Pm-Scl
RNA-Polymerase 1,2 und 3
Th
Jo-1
Mi-2
PL7
PL12
SRP.

6. Verfahren gemäß Anspruch 1, wobei die Mehrzahl von Autoantikörpern Antikörper zu jedem der folgenden Antigene umfasst:
SSA 60
SSA 60
SSA 52
SSB 48
Sm BB¹
Sm D1
RNP 68
RNP A
RNP C
Fibrillarin
Riboproteine P0, P 1 und P2
dsDNA
Nukleosom
Ku
Zentromer A
Zentromer B
Scl-70
Pm-Scl
RNA-Polymerase 1,2 und 3
Th
Jo-1
Mi-2
PL7
PL12
SRP.

7. Verfahren gemäß Anspruch 1, wobei die Bibliothek der Bezugsdatensätze 100 bis 10000 biologische Proben von Bezugspersonen darstellt, von denen bekannt ist, dass sie systemische Autoimmunerkrankungen bekannter Identität haben.

8. Verfahren gemäß Anspruch 1, wobei die Bibliothek der Bezugspersonen 200 bis 2000 biologische Proben von Bezugspersonen darstellt, von denen bekannt ist, dass sie systemische Autoimmunerkrankungen bekannter Identität haben.

9. Verfahren gemäß Anspruch 1, wobei der Schritt (b) ferner das Zuweisen eines Sicherheitsgrades an die Bestimmung umfasst.

10. Verfahren gemäß Anspruch 1, wobei die biologische Probe der Testperson ein Mitglied ist, das ausgewählt ist aus der Gruppe bestehend aus Serum, Plasma, Urin und zerebrospinaler Flüssigkeit.

11. Verfahren gemäß Anspruch 1, wobei die biologische Probe der Testperson Serum ist.

12. Verfahren gemäß Anspruch 1, wobei der Schritt (a) durch Immunassay durchgeführt wird.

13. Verfahren gemäß Anspruch 1, wobei der Schritt (a) durch Immunassay mit Fluoreszenzdetektion durchgeführt wird.

14. Verfahren gemäß Anspruch 1, wobei einer der systemischen Autoimmunerkrankungen systemischer Lupus Erythematosus ist.

## Revendications

1. Procédé mis en oeuvre par ordinateur pour l'identification d'une maladie auto-immune systémique chez un sujet à tester suspecté de souffrir d'une ou plusieurs maladies auto-immunes systémiques non identifiées autrement, sélectionnées dans le groupe consistant en lupus érythémateux systémique, sclérodermie, syndrome de Sjögren, polymyositis, dermatomyositis, CREST, et maladie du tissu conjonctif mixte, ledit procédé comprenant :
(a) la réception d'un jeu de données de test pour le sujet à tester, lequel jeu de données de test est obtenu à partir d'un échantillon biologique du sujet à tester et inclut des valeurs représentant des niveaux d'une pluralité d'anticorps ;
(b) la comparaison du jeux de données de test avec une bibliothèque de jeux de données de référence en appliquant l'analyse du k-plus proche voisin pour produire une décision statistiquement dérivée indiquant si à partir de la plage pour aucune, une ou plusieurs desdites maladies auto-immunes systémiques, le sujet à tester souffre d'une ou de plusieurs desdites maladies auto-immunes systémiques ou d'aucune d'entre elles, les jeux de données de référence ayant été obtenus par le stockage d'une pluralité de jeux de données de références incluant i) les jeux de données de référence obtenus par la soumission d'échantillons biologiques de sujets de référence, chacun ayant une desdites une ou plusieurs des maladies auto-immunes systémiques, à un jeu d'un ou plusieurs tests, et ii) les jeux de données de référence obtenus par la soumission des échantillons biologiques des sujets de référence connus pour ne pas avoir une desdites une ou plusieurs des maladies auto-immunes systémiques audit jeu d'un ou de plusieurs tests, de sorte que chaque jeu de données de référence stocké inclut des valeurs représentant des niveaux de la pluralité d'anticorps et chaque jeu de données de référence stocké est associé à une ou plusieurs desdites maladies auto-immunes systémiques ou à aucune d'entre elles; et
(c) l'identification de celle desdites maladies auto-immunes systémiques dont le sujet à tester souffre si la décision statistiquement dérivée indique que le sujet à tester souffre d'une ou de plusieurs desdites maladies auto-immunes systémiques.

2. Procédé selon la revendication 1, dans lequel ledit sujet à tester souffre de deux maladies auto-immunes systémiques, et l'étape (b) comprend l'application de l'analyse du k-plus proche voisin pour produire une décision statistiquement dérivée indiquant les deux maladies auto-immunes systémiques dont souffre le sujet à tester.

3. Procédé selon la revendication 1, dans lequel ladite pluralité d'anticorps compte de 10 à 100 anticorps.

4. Procédé selon la revendication 1, dans lequel ladite pluralité d'anticorps compte de 15 à 25 anticorps.

5. Procédé selon la revendication 1, dans lequel ladite pluralité d'anticorps comprend des anticorps d'au moins quinze des antigènes suivants :
SSA 60
SSA 60
SSA 52
SSB 48
Sm BB¹
Sm D1
RNP 68
RNP A
RNP C
Fibrillarine
Riboprotéines P0, P1 et P2
ADN double brin
Nucléosome
Ku
Centromère A
Centromère B
Scl-70
Pm-Scl
ARN-Polymérasesl,2 et3
Th
Jo-1
Mi-2
PL7
PL12
SRP.

6. Procédé selon la revendication 1, dans lequel ladite pluralité d'anticorps comprend des anticorps de chacun des antigènes suivants :
SSA 60
SSA 60
SSA 52
SSB 48
Sm BB¹
Sm D1
RNP 68
RNP A
RNP C
Fibrillarine
Riboprotéines P1, P1 et P2
ADN double brin
Nucléosome
Ku
Centromère A
Centromère B
Scl-70
Pm-Scl
ARN-Polymérases 1,2 et3
Th
Jo-1
Mi-2
PL7
PL12
SRP.

7. Procédé selon la revendication 1, dans lequel ladite bibliothèque de jeux de données de référence représente de 100 à 10000 échantillons biologiques provenant des sujets de référence connus pour avoir des maladies auto-immunes systémiques d'identité connue.

8. Procédé selon la revendication 1, dans lequel ladite bibliothèque de jeux de données de référence représente de 200 à 2000 échantillons biologiques provenant de sujets de référence connus pour avoir des maladies auto-immunes systémiques d'identité connue.

9. Procédé selon la revendication 1, dans lequel l'étape (b) comprend en outre l'attribution d'un niveau de confiance à ladite détermination.

10. Procédé selon la revendication 1, dans lequel ledit échantillon biologique provenant dudit sujet à tester est un membre sélectionné dans le groupe consistant en sérum, plasma, urine, et liquide céphalo-rachidien.

11. Procédé selon la revendication 1, dans lequel ledit échantillon biologique provenant du sujet à tester est du sérum.

12. Procédé selon la revendication 1, dans lequel l'étape (a) est réalisée par essai immunologique.

13. Procédé selon la revendication 1, dans lequel l'étape (a) est réalisée par essai immunologique avec détection par fluorescence.

14. Procédé selon la revendication 1, dans lequel ladite maladie auto-immune systémique est un lupus érythémateux systémique.
